# EUROPEAN PATENT SPECIFICATION

(11) **EP 3 601 352 B1**
(45) Date of publication and mention of the grant of the patent: **17.02.2021**
(21) Application number: 18718631.7
(22) Date of filing: 21.03.2018
(51) Int. Cl.: C07K 16/28, A61P 31/06, A61K 39/00, A61K 39/395

(54) **TYPE 1 INTERFERON RECEPTOR ANTAGONISTS FOR USE IN METHODS OF TREATING TUBERCULOSIS AND OTHER INFECTIOUS DISEASES**
TYP-1-INTERFERONREZEPTORANTAGONISTEN ZUR VERWENDUNG BEI VERFAHREN ZUR BEHANDLUNG VON TUBERKULOSE UND ANDEREN INFEKTIONSKRANKHEITEN
ANTAGONISTES DU RÉCEPTEUR DE L'INTERFÉRON DE TYPE 1 DESTINÉS À ÊTRE UTILISÉS DANS DES PROCÉDÉS DE TRAITEMENT DE LA TUBERCULOSE ET D'AUTRES MALADIES INFECTIEUSES

(30) Priority: 21.03.2017 US 201762474229 P; 25.07.2017 US 201762536696 P
(43) Date of publication of application: 05.02.2020
(73) Proprietor: Austrianni GmbH, 1030 Vienna (AT)
(72) Inventor: WABL, Matthias, San Francisco, California 94122 (US); ADOLF, Günther, 1070 Vienna (AT)
(74) Representative: Goodfellow, Hugh Robin
(86) International application number: PCT/IB2018/051900
(87) International publication number: WO 2018/172957

(56) References cited:
- WO-A2-2009/100309
- RU-C1- 2 228 197
- FINLAY W. MCNAB ET AL: "Type I IFN Induces IL-10 Production in an IL-27-Independent Manner and Blocks Responsiveness to IFN-[gamma] for Production of IL-12 and Bacterial Killing in Mycobacterium tuberculosis -Infected Macrophages", THE JOURNAL OF IMMUNOLOGY, vol. 193, no. 7, 1 October 2014 (2014-10-01), pages 3600-3612, XP055476369, US ISSN: 0022-1767, DOI: 10.4049/jimmunol.1401088
- LÚCIA MOREIRA-TEIXEIRA ET AL: "Type I interferons in tuberculosis: Foe and occasionally friend", THE JOURNAL OF EXPERIMENTAL MEDICINE, vol. 215, no. 5, 17 April 2018 (2018-04-17), pages 1273-1285, XP055476392, US ISSN: 0022-1007, DOI: 10.1084/jem.20180325
- MEG L. DONOVAN ET AL: "Type I Interferons in the Pathogenesis of Tuberculosis: Molecular Drivers and Immunological Consequences", FRONTIERS IN IMMUNOLOGY, vol. 8, 27 November 2017 (2017-11-27), XP055476183, DOI: 10.3389/fimmu.2017.01633
- KATRIN D. MAYER-BARBER ET AL: "Host-directed therapy of tuberculosis based on interleukin-1 and type I interferon crosstalk", NATURE, vol. 511, no. 7507, 25 June 2014 (2014-06-25), pages 99-103, XP055476229, GB ISSN: 0028-0836, DOI: 10.1038/nature13489

### SEQUENCE LISTING

<110> AUSTRIANNI GMBH
<120> Type 1 interferon receptor antagonists for use in methods of treating tuberculosis and other infectious diseases
<130> P072646WO
<141> 2018-03-21
<160> 10
<170> SeqWin2010, version 1.0
<210> 1
   <211> 7
   <212> PRT
   <213> Artificial Sequence
<220>
   <223> HCDR1 for anifrolumab
<400> 1
   Gly Tyr Ile Phe Thr Asn Tyr
   1               5
<210> 2
   <211> 6
   <212> PRT
   <213> Artificial Sequence
<220>
   <223> HCDR2 for anifrolumab
<400> 2
   Tyr Pro Gly Asp Ser Asp
   1               5
<210> 3
   <211> 8
   <212> PRT
   <213> Artificial Sequence
<220>
   <223> HCDR3 for anifrolumab
<400> 3
   His Asp Ile Glu Gly Phe Asp Tyr
   1               5
<210> 4
   <211> 12
   <212> PRT
   <213> Artificial Sequence
<220>
   <223> LCDR1 for anifrolumab
<400> 4
   Arg Ala Ser Gln Ser Val Ser Ser Ser Phe Phe Ala
   1               5                   10
<210> 5
   <211> 7
   <212> PRT
   <213> Artificial Sequence
<220>
   <223> LCDR2 for anifrolumab
<400> 5
   Gly Ala Ser Ser Arg Ala Thr
   1               5
<210> 6
   <211> 9
   <212> PRT
   <213> Artificial Sequence
<220>
   <223> LCDR3 for anifrolumab
<400> 6
   Gln Gln Tyr Asp Ser Ser Ala Ile Thr
   1               5
<210> 7
   <211> 117
   <212> PRT
   <213> Artificial Sequence
<220>
   <223> Heavy chain variable region for anifrolumab
<400> 7
<210> 8
   <211> 108
   <212> PRT
   <213> Artificial Sequence
<220>
   <223> Light chain variable region for anifrolumab
<400> 8
<210> 9
   <211> 447
   <212> PRT
   <213> Artificial Sequence
<220>
   <223> Heavy chain for anifrolumab
<400> 9
   Glu Val Gln Leu Val Gln Ser Gly Ala Glu Val Lys Lys Pro Gly Glu
   1               5                   10                  15
   Ser Leu Lys Ile Ser Cys Lys Gly Ser Gly Tyr Ile Phe Thr Asn Tyr
               20                  25                  30
   Trp Ile Ala Trp Val Arg Gln Met Pro Gly Lys Gly Leu Glu Ser Met
           35                  40                  45
   Gly Ile Ile Tyr Pro Gly Asp Ser Asp Ile Arg Tyr Ser Pro Ser Phe
       50                  55                  60
   Gln Gly Gln Val Thr Ile Ser Ala Asp Lys Ser Ile Thr Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg His Asp Ile Glu Gly Phe Asp Tyr Trp Gly Arg Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Arg Val Glu Pro Lys Ser Cys Asp Lys Thr His
       210                 215                 220
   Thr Cys Pro Pro Cys Pro Ala Pro Glu Phe Glu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Ser Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Tyr Ser Lys Leu Thr Val Asp Lys Ser Arg
                   405                 410                 415
   Trp Gln Gln Gly Asn Val Phe Ser Cys Ser Val Met His Glu Ala Leu
               420                 425                 430
   His Asn His Tyr Thr Gln Lys Ser Leu Ser Leu Ser Pro Gly Lys
           435                 440                 445
<210> 10
   <211> 215
   <212> PRT
   <213> Artificial Sequence
<220>
   <223> Light chain for anifrolumab
<400> 10
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Phe Phe Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Leu Ser
       50                  55                  60
   Gly Ser Gly Ser Gly Thr Asp Phe Thr Leu Thr Ile Thr Arg Leu Glu
   65                  70                  75                  80
   Pro Glu Asp Phe Ala Val Tyr Tyr Cys Gln Gln Tyr Asp Ser Ser Ala
                   85                  90                  95
   Ile Thr Phe Gly Gln Gly Thr Arg Leu Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215

## Description

### CROSS-REFERENCE

This application is related to U.S. Provisional Patent Application No. 62/474,229, filed 21st March 2017, and U.S. Provisional Patent Application No. 62/536,696, filed 25th July 2017.

### FIELD OF THE INVENTION

The present invention relates to the use of type 1 interferon receptor antagonists in methods of treating tuberculosis and other infectious diseases, in which type 1 interferon signalling has been found to be deleterious.

### BACKGROUND TO THE INVENTION

Type 1 interferons (type 1 IFNs), which comprise IFN-alpha, IFN-beta, IFN-omega (as well as several others, depending on the species of interest), are a family of structurally related cytokines having antiviral, antitumor and immunomodulatory effects [1]. The human IFN-alpha locus includes two subfamilies. The first subfamily consists of at least 14 non allelic genes and 4 pseudogenes having at least 75% homology. The second subfamily, alpha-II or omega, contains 5 pseudogenes and 1 functional gene which exhibits 70% homology with the IFN-alpha genes. The subtypes of IFN-alpha have different specific activities but they possess the same biological spectrum and have the same cellular receptor.

All human type 1 IFNs bind to a cell surface receptor (IFN alpha receptor, IFNAR) consisting of two transmembrane proteins, IFNAR-1 and IFNAR-2 [2,3]. IFNAR-1 is essential for high-affinity binding and differential specificity of the IFNAR complex [1]. While no functional differences have been identified, it is thought that each type 1 IFN subtype exhibits different interactions with the IFNAR receptor components leading to potentially diverse signalling outcomes [4]. In particular, studies utilizing mutant forms of IFNAR1 and IFNAR2 suggested that alpha- and beta-interferons signal differently through the receptor by interacting differentially with its respective chains [5].

Binding to the IFN-alpha receptor results in the activation of intracellular signal transduction pathways [6], initiated by the activation of the Jak kinases, Jak1 and Tyk2. These kinases subsequently phosphorylate signal transducer and activator of transcription (STAT) proteins, STAT1 and STAT2. Phosphorylated STAT proteins, together with p48, form the transcription factor complex IFN-stimulated gene factor 3 (ISGF3) that translocates into the nucleus. The complex binds IFN-stimulated response elements (ISREs) and induces the expression of IFN-inducible genes.

Type 1 IFNs are part of the innate immunological defence and are produced after introduction of foreign substances into the body. In particular, viruses have the ability to evoke production of type 1 IFNs, but bacteria, fungi and other "non-self' agents may also induce type 1 IFN production. Indeed, the type 1 IFN signalling pathway plays an important role in the immune defence against viral and certain bacterial infections.

Early functional studies of type 1 IFNs have focused on their involvement in the innate defence against viral infections [7]. More recent studies have implicated type 1 IFNs as potent immunoregulatory cytokines in the adaptive immune response. Specifically, type 1 IFNs have been shown to facilitate differentiation of naive T cells along the Th1 pathway [8], to enhance antibody production [9] and to support the functional activity and survival of memory T cells [10].

Lately, it has been recognized that type 1 IFN signalling may be deleterious in a variety of bacterial, parasitic and fungal infections, including tuberculosis, however, these effects have not been universally observed. Importantly, these considerations have not resulted in the discovery of any new therapeutic approaches towards the treatment of these infections. Finlay et al., (The Journal of Immunology, 2014,; 193:3600-3612) report that type I interferon is a negative regulator of the immune response to M. tuberculosis. The present disclosure shows for the first time that a type 1 interferon receptor antagonist can be used for the treatment of infectious diseases in which type 1 IFN signalling has been found to be deleterious. The inventors have surprisingly found that antibodies directed against the type 1 IFN receptor, when administered to mammals such as mice or monkeys infected with *Mycobacterium tuberculosis,* are able to suppress bacterial replication, reduce tissue damage and prolong survival of the infected animals.

### SUMMARY OF THE INVENTION

The invention provides a type I interon receptor antagonist which specifically binds to IFNAR1, inhibits signalling of type 1 IFNs and is an anti-type 1 interferon receptor antibody or an antigen-binding fragment thereof for use in a method of treating a subject suffering from an infectious disease in which type 1 IFN signalling is detrimental to the subject or promotes disease progression.

In some embodiments, the anti-type 1 interferon receptor antibody is a polyclonal, monoclonal, multispecific, mouse, human, humanized, primatized or chimeric antibody or a single-chain antibody. In one specific embodiment, the anti-type 1 interferon receptor antibody is human or humanized. In another specific embodiment, the anti-type 1 interferon receptor antibody is a monoclonal antibody. In some embodiments, the anti-type 1 interferon receptor antibody or antigen-binding fragment thereof is selected from a Fab, Fab', F(ab')2, Fd, Fv, a single-chain Fv (scFv) and a disulfide-linked Fv (sdFv).

In some embodiments, the infectious disease is a bacterial infectious disease. In a specific embodiment, the infectious disease is caused by any one of *Mycobacterium spp., Francisella spp., Brucella spp., Candida spp., Cryptococcus spp., Chlamydia spp., Escherichia spp., Helicobacter spp., Listeria spp., Legionella spp., Staphylococcus spp.; Shigella spp., Streptococcus spp., Salmonella spp., Tropheryma spp.* or *Yersinia spp.*

In a specific embodiment, the infectious disease is tuberculosis. In some embodiments, the infectious disease is caused by an agent selected from *Mycobacterium tuberculosis, M. africanum, M. bovis, M. canetti, M. caprae, M. microti, M. mungi, M. orygis, M. pinnipedii, M. suricattae, M. kansasii, M. xenopi, M. scrofulaceum, M. abscessus, M. haemophilum* or *M. ulcerans.* In other embodiments, the tuberculosis is drug-resistant tuberculosis. In certain embodiments, the drug resistant tuberculosis is resistant to at least one of isoniazid, rifampicin, bedaquiline, delamanid, pyrazinamide and ethambutol. In other embodiments, the tuberculosis is multidrug-resistant tuberculosis and is resistant to at least one of isoniazid or rifampicin and at least one other anti-mycobacterial agent.

In another specific embodiment, the infectious disease is caused by *Francisella.* In some embodiments, the infectious disease is caused by an agent selected from *Francisella tularensis, F. novicida, F. hispaniensis, F. persica comb. nov., F. noatunensis, F. philomiragia, F. halioticida, F. endociliophora, F. guangzhouensis* or *F. piscicida.*

In a further specific embodiment, the infectious disease is caused by a kinetoplastid. In some embodiments, the infectious disease is leishmaniasis. In a specific embodiment, the infectious disease is caused by an agent selected from *Leishmania major, L. aethiopica, L. amazonensis, L. arabica, L. archibaldi, L. aristedesi, L. (Viannia) braziliensis, L. chagasi (syn. L. infantum), L. (Viannia) colombiensis, L. deanei, L. donovani, L. enriettii, L. equatorensis, L. forattinii, L. garnhami, L. gerbili, L. (Viannia) guyanensis, L. herreri, L. hertigi, L. infantum, L. killicki, L. (Viannia) ainsoni, L. mexicana, L. (Viannia) naiffi, L. (Viannia) panamensis, L. (Viannia) peruviana,* , *L. (Viannia) pifanoi, L. (Viannia) shawi, L. tarentolae, L. tropica, L. turanica* or *L. venezuelensis.*

In other embodiments, the infectious disease is African trypanosomiasis or sleeping sickness and the causative agent is *Trypanosoma brucei.* In a specific embodiment, the infectious disease is caused by *T. brucei gambiense* or *T. brucei rhodesiense.* In yet another embodiment, the infectious disease is Chagas disease and the causative agent is *Trypanosoma cruzi.*

In some embodiments, the type 1 interferon receptor antagonist is administered by intravenous, intramuscular, intraperitoneal, intracerobrospinal, subcutaneous, intra-articular, intrasynovial, intrathecal, oral or topical administration or by inhalation. In a specific embodiment, the type 1 interferon receptor antagonist is administered by intravenous or subcutaneous administration.

In some embodiments, the type 1 interferon receptor antagonist is administered at a dosage of about 0.01 mg/kg to about 100 mg/kg of the subject's body weight. In a specific embodiment, the type 1 interferon receptor antagonist is administered at a dosage of about 10 mg/kg to about 30 mg/kg of the subject's body weight. In another specific embodiment, the type 1 interferon receptor antagonist is administered at a dosage of about 0.3 mg/kg body weight; about 1 mg/kg body weight; about 3 mg/kg body weight; or about 10 mg/kg body weight. In a further specific embodiment, the type 1 interferon receptor antagonist is administered at a dosage of about 10 mg/kg body weight; about 15 mg/kg body weight; about 20 mg/kg body weight; about 25 mg/kg body weight; or about 30 mg/kg body weight.

In some embodiments, the type 1 interferon receptor antagonist is administered at a fixed dosage. In a specific embodiment, the type 1 interferon receptor antagonist is administered at a fixed dosage from about 50 mg to about 2000 mg, for example about 50 mg to about 1500 mg, about 50 mg to about 1000 mg, about 50 mg to about 500 mg, about 50 mg to about 100 mg, about 100 mg to about 2000 mg, about 200 mg to about 2000 mg, about 500 mg to about 2000 mg, about 1000 mg to about 2000 mg, about 1500 mg to about 2000 mg, about 100 mg to about 1500 mg, about 200 mg to about 1500 mg, about 500 mg to about 1500 mg, about 750 mg to about 1500 mg, about 1000 mg to about 1500 mg, about 700 mg to about 1200 mg, about 800 mg to about 1200 mg or about 900 mg to about 1100 mg. In certain embodiments, the type 1 interferon receptor antagonist is administered at a dosage of: about 100 mg; about 200 mg; about 300 mg; about 600 mg; about 1000 mg; about 1200 mg; about 1500 mg; or about 2000 mg.

In some embodiments, the type 1 interferon receptor antagonist is administered as a single dose; in two or more doses once per week; once every two weeks; once every three weeks; once every four weeks; once a month; once every 3 months; or once every six months.

In some embodiments the type 1 interferon receptor antagonist is administered in intervals of one day to six months. In a specific embodiment, the type 1 interferon receptor antagonist is administered in intervals of 1 week; 2 weeks; 3 weeks; 4 weeks; 1 month; 2 months; 3 months; 4 months; 5 months; or 6 months.

In one particular embodiment, the type 1 interferon receptor antagonist is administered intravenously or subcutaneously at doses of 1 mg/kg to 30 mg/kg of body weight, at intervals of 1 week to 4 weeks.

In some embodiments, methods of the invention comprise further administering to the subject a therapeutically effective amount of an antimicrobial or antibacterial agent. In a specific embodiment, the antimicrobial or antibacterial agent is selected from isoniazid, rifampicin, bedaquiline, delamanid, pyrazinamide or ethambutol.

### DESCRIPTION OF FIGURES

FIGURE 1: Plots showing the colony forming unit (CFU) counts in the bronchoalveolar lavage (BAL), lung, spleen and liver fractions of treated and untreated mice 21 and 34 days post-infection with *Mycobacterium tuberculosis* strain H37Rv.
FIGURE 2: The disease-score of treated and untreated groups of mice at various time points post-infection with Mycobacterium tuberculosis strain H37Rv.

### DETAILED DESCRIPTION OF THE INVENTION

The present disclosure relates to the use of a type 1 interferon receptor antagonist, in particular an anti-type 1 interferon receptor antibody, in the treatment of infectious diseases, in which type 1 IFN signalling has been found to be deleterious, e.g. in situations where the production or expression of type 1 IFNs is associated with a worsening of disease symptoms. Typically the infectious disease is not a viral infectious disease.

At first sight, it may appear counterintuitive to target an important innate immunological defence mechanism to treat infectious diseases. However, recent clinical studies have shown that anti-type 1 interferon receptor antibodies can be used safely in the therapy of autoimmune diseases without causing severe or uncontrollable immunosuppression [11]. Therefore, without being bound by any particular theory, the inventors believe that suppression of type 1 signalling by a type 1 interferon receptor antagonist in infectious diseases where such signalling is detrimental will be beneficial for patients suffering from these infectious diseases without unduly compromising their ability to respond to infections, in particular those caused by viruses.

Specifically, a recent clinical trial with the anti-type 1 interferon receptor antibody anifrolumab did not increase the incidence of serious adverse events [11]. Furthermore the trial investigators found that diminished responses to certain viral infections were manageable with anti-viral treatments. In that trial, 305 patients suffering from systemic lupus erythematosus were randomized and either received intravenous anifrolumab (300 mg or 1,000 mg) or placebo every 4 weeks for 48 weeks, in addition to standard-of-care medications. Both herpes zoster and influenza infections were slightly more frequent in the anifrolumab-treatment group. However, all patients responded promptly to antiviral treatment. The percentages of patients reported as developing influenza were greater in the anifrolumab 300 mg (6.1%) and 1,000 mg (7.6%) groups than in the placebo group (2.0%). Similarly, upper respiratory infections occurred more frequently in patients treated with anifrolumab (300 mg: 36.4%; 1,000 mg: 41.9%) compared with placebo (28.7%). However, the slight increase in viral infections did not increase the incidence of serious adverse events and these infections were also manageable with anti-viral treatments.

### Tuberculosis

Tuberculosis (TB) is a potentially life-threatening condition affecting the lungs as well as other organs. The disease is caused by transmission of an infectious agent, *Mycobacterium tuberculosis* (*Mtb*), from a patient with active lung disease. Bacteria in the patient's sputum are aerosolized by coughing and inhaled by bystanders. The extremely high prevalence of TB is often underestimated: according to WHO estimates, about 2 billion people are infected. The vast majority of these patients do not require treatment and do not transmit the infection, as their immune system is able to suppress bacterial replication and spreading ("latent" infection), but they carry a lifetime risk of about 10% to develop active disease. About 10 million people annually are diagnosed with active TB. As a consequence of lack of treatment or resistance to available drugs about 1.8 million patients die each year.

Current therapeutic regimes for treatment-naive patients are based on a combination of several chemotherapeutic agents, to be administered for at least 6 months. First-line therapy is able to cure about 85-90% of these patients, however, 10-15% eventually relapse as their bacteria have developed drug resistance (estimated number of cases per year: 580,000). Treatment of patients with multidrug resistant TB (MDR-TB) is more complex, lasts up to 2 years and may involve hospitalization for prolonged periods of time. Further, treatment may result in severe toxicity, is able to cure only about 50% of patients and is associated with considerable cost to healthcare providers. Although TB thus is a disease with high unmet medical need, the current portfolio of new therapeutic agents in clinical development is very small, comprising only 8 drug candidates. All of these are low-molecular-mass, chemically synthesized compounds. The portfolio currently does not include any monoclonal antibody drugs.

Studies performed in both patients and mouse models of infection collectively point to a detrimental role of IFNα/β during tuberculosis. Several studies have reported a decreased bacterial load and/or improved host survival in the absence of IFNα/β-mediated signalling [12, 13, 14, 15]. However, these changes have not been universally observed [16], and there has not always been concordance between studies regarding bacterial load and survival data. It is likely that the differences between studies result from differences in experimental protocols, and in the genetics of the host and the *M. tuberculosis* strain used.

The importance of type 1 IFNs as a detrimental factor during tuberculosis of humans was first observed in studies of patient cohorts from the United Kingdom and South Africa [17]. Patients with active tuberculosis had a prominent whole blood IFNα/β-inducible transcriptional profile that correlated with the extent of radiographic disease and diminished with successful treatment [17]. Several other studies have since verified these findings in additional patient cohorts from Africa [18, 19] and Indonesia [20], suggesting that this IFNα/β-inducible signature is broadly applicable to humans.

IFNα/β overexpression during *M*. *tuberculosis* infection in experimental mouse models has provided additional evidence for the detrimental effects of the IFNα/β system during tuberculosis. Studies of infection with hyper-virulent *M. tuberculosis* strains showed a correlation between increased levels of IFNα/β and increased virulence [12, 13, 15]. Direct instillation of IFNα/β into the lungs during infection was also injurious to the host [15]. Similarly, enhanced induction of IFNα/β expression during *M. tuberculosis* infection via administration of a TLR3 ligand derivative led to an increased severity of infection [21, 22]. Deletion of the gene encoding a negative regulator of IPNα/β, MAPK kinase kinase 8 (MAP3K8; also known as TPL2), which functions downstream of TLRs, led to increased levels of IFNα/β production and increased bacterial burdens [23]. These increases were abrogated in *Map3k8*^{*-*/}*⁻Ifnar1*^{*-*/*-*} (double knockout) mice during *M. tuberculosis* infection. Control of the bacterial load in *Map3k8*^{*-*/}*⁻Ifnar1*^{-/-} mice was correlated with reduced IL-10 levels and increased IL-12 levels in the serum. It has further been observed that concurrent infection of mice with *M. tuberculosis* and influenza A virus, which induces IFNα/β expression, resulted in increased bacterial loads in an IFNα/β-dependent manner [24].

The mechanisms that mediate the IFNα/β-driven exacerbation of TB are not fully understood but seem to be multifactorial. Data from investigations of hyper-virulent *M. tuberculosis* strains initially suggested that the suppression of pro-inflammatory cytokines and of TH1-type Immunity are important [12, 13, 15]. There is good evidence both in human cells and in mouse models that IFNα/β signalling suppresses the production of host-protective cytokines following *M*. *tuberculosis* infection. The production of IL-1α and IL-1β, which are crucial for host defence against *M. tuberculosis* [25], is inhibited by IFNα/β both *in vitro* in infected human and mouse cells and *in vivo* in mouse models [22, 26, 27, 28]. The induction of the immunosuppressive cytokines IL-10 and IL-1 receptor antagonist (IL1RA) by IFNα/β seems to play an important role in the suppression of pro-inflammatory cytokines [22, 23, 26, 28]. The production of other pro-inflammatory cytokines such as TNF and IL-12 is also negatively affected [23, 26, 28].

In both mouse and human cells, it has been shown that IFNα/β potently suppress the ability of macrophages to upregulate anti-mycobacterial effector molecules and to restrict bacterial growth in response to both *Mycobacterium leprae* and *M. tuberculosis* [26]. Cell-intrinsic type 1 IFN signals have been shown to negatively regulate iNOS production by pulmonary myeloid cells, particularly TIP-DCs [22].

The inventors have recognized that these insights offer novel opportunities for therapeutic intervention using a type 1 interferon receptor antagonist which aims at activating the immune system of TB patients and increasing cure rates, prolongation of survival and/or progression-free survival. Type 1 interferon receptor antagonist therapy will also be useful for the treatment of patients with latent TB, particularly those with subclinical disease at high risk of progression.

Without being bound by any particular theory, the inventors believe that the use of a type 1 interferon receptor antagonist according to the invention is useful to: (i) reduce and/or delay the formation or severity of TB lesions, (ii) reduce and/or delay the dissemination of mycobacteria to lymph nodes and other organs, (iii) extend survival of the infected subject, and/or (iv) enhance the efficacy of other drugs used to treat infection. Indeed, an important goal of the invention is to delay the progress of the infection and prevent damage to tissues and organs in order to allow antimicrobial chemotherapeutic agents to bring the infection under control.

Accordingly, in one aspect of the invention, a method of treating tuberculosis in a human subject is provided, said method comprising administering to the subject a type 1 interferon receptor antagonist. In some embodiments, the invention provides a method of treating one or more of the symptoms of tuberculosis, which include coughing, coughing up blood, chest pain, painful breathing, weight loss, fatigue, fever, sweating, chills and loss of appetite.

The severity, progression, response to treatment, and other clinical measures of the symptoms of tuberculosis normally include chest x-rays, sputum tests and skin tests (e.g. tuberculin test). Accordingly, in some embodiments, the disclosure provides a method of treating tuberculosis in a patient in need of such treatment, comprising administering a therapeutically effective amount of a type 1 interferon receptor antagonist, wherein the treatment causes an improvement in the patient's chest x-ray, sputum test or skin test (e.g. tuberculin).

The type 1 interferon receptor antagonist may be used as a single agent or in combination with anti-mycobacterial agents currently used to treat patients with drug-sensitive TB, such as isoniazid or rifampicin, as well as patients with multidrug-resistant disease, such as bedaquiline or delamanid.

The type 1 interferon receptor antagonist of the present invention is also useful for treating patients suffering from infections with other pathogens of the *Mycobacterium tuberculosis* complex, such as *Mycobacterium bovis* or *Mycobacterium africanum,* or of infections with "atypical" mycobacteria that result in significant morbidity and mortality particularly in the elderly population, such as *Mycobacterium avium* complex, *Mycobacterium abscessus* or *Mycobacterium ulcerans.*

Accordingly, in some embodiments, the mycobacterial infection that can be treated using methods of the invention is caused by *M. tuberculosis, M. africanum, M. bovis, M. bovis BCG, M. canetti, M. caprae, M. microti, M. mungi, M. orygis, M. pinnipedii, M. suricattae, M. kansasii, M. xenopi, M. scrofulaceum, M. abscessus, M. haemophilum* or *M. ulcerans.*

In some embodiments, the *Mycobacterium tuberculosis* to be treated in accordance with the present invention is one of the following *Mycobacterium tuberculosis* strains: H37Rv, BTB05-552, BTB05-559, CDC1551, CTRI-2, F11, H37, H37Ra, HN878, KZN 1435, KZN 4207, KZN R506, KZN V2475, R1207, RGTB327, S96-129, X122, '98-R604 INH-RIF-EM', 02_1987, 210, 94_M4241A, C, CDC1551A, CPHL_A, CTRI-4, EAS054, GM 1503, K85, KZN 605, OSDD071, OSDD504, OSDD518, SUMu001, SUMu002, SUMu003, SUMu004, SUMu005, SUMu006, SUMu007, SUMu008, SUMu009, SUMu010, SUMu011, SUMu012, T17, T46, T85, T92, W-148, str. Haarlem, 210_16C10, 210_16C2_24C1, 210_16C2_24C2, 210_32C4, 210_4C15, 210_4C15_16C1, 210_4C15_16C1_48C1, 210_4C15_16C1_48C2, 210_4C15_16C1_56C1, 210_4C15_16C1_56C2, 210_4C31, 210_4C31_16C1, 210_4C31_16C1_24C1, 210_4C31_16C1_40C1, 210_4C31_16C2, 210_8C1, 210_8C6, BC, CTRI-3, H37Rv_2009, NJT210GTG, str. Erdman=ATCC 35801, str. Erdman WHO, CCDC5079, CCDC5180, RGTB423, UT205, CTRI-1, H37RvAE, H37RvCO, H37RvHA, H37RvJO, H37RvLP, H37RvMA, LAM7, NCGM2209, RGTB306, WX1, WX3, XDR1219, XDR1221, str. Beijing/W BT1, or str. Erdman (ATCC 35801).

### Other infectious diseases

A method of treating infectious diseases other than mycobacterial infections is provided, wherein said method comprises administering to a subject a type 1 interferon receptor antagonist. The method is particularly suitable in situations where the production or expression of type 1 IFNs is associated with a worsening of pathological symptoms.

The infectious disease that can be treated include Lyme disease, granuloma inguinale, bacterial vaginosis, gonorrhea, syphilis, melioidosis, anthrax, leptospirosis, whooping cough, leprosy, tetanus, plague, bubonic plague, pneumonic plague, scarlet fever, streptococcal infections, meningococcal disease, bacteremia, cholera, dysentery, amebic dysentery, shigellosis, diphtheria, Legionnaires' disease, *Hemophilus influenzae,* typhoid fever, Rocky Mountain spotted fever, *Vibrioparahaemolyticus, Vibrio vulnificu,* Yersiniosis, Whipple's disease, bacterial digestive infections, meningitis, encephalitis, impetigo, cellulitis, sepsis, septicemia, pneumonia, *Salmonella* infection, staphylococcal infection, botulism, *Escherichia coli* infection, rheumatic fever, *Legionella* infection, *Penicillium* infection, bacterial conjunctivitis, pneumococcal meningitis, bacterial septicemia, chlamydia, mastitis and pseudomonas infections.

Infectious disease that can be treated is caused by infection with one of *Brucella, Candida, Cryptococcus, Chlamydia, Escherichia, Francisella, Helicobacter, Leishmania, Listeria, Legionella,, Plasmodium, Staphylococcus; Shigella, Streptococcus, Salmonella, Tropheryma* or *Yersinia.* In a particular embodiment, the infection is caused by *Brucella abortus, Candida albicans, Cryptococcus neoformans, Chlamydia pneumoniae, Chlamydia trachomatis, Escherichia coli, Francisella tularensis subsp. novicida, Helicobacter pylori, Leishmania major, Listeria monocytogenes, Legionella pneumophila, Plasmodium berghei, Staphylococcus aureus, Shigella flexneri, Streptococcus pneumoniae, Salmonella enterica subsp. enterica serovar Typhimurium, Tropheryma whipplei,* and *Yersinia pestis.*

The infectious disease that can be treated is leishmaniasis. The leishmania infection is caused by an agent selected from *Leishmania major, L. aethiopica, L. amazonensis, L. arabica, L. archibaldi, L. aristedesi, L. (Viannia) braziliensis, L. chagasi (syn. L. infantum), L. (Viannia) colombiensis, L. deanei, L. donovani, L. enriettii, L. equatorensis, L. forattinii, L. garnhami, L. gerbili, L. (Viannia) guyanensis, L. herreri, L. hertigi, L. infantum, L. killicki, L. (Viannia) ainsoni, L. mexicana, L. (Viannia) naiffi, L. (Viannia) panamensis, L. (Viannia) peruviana, , L. (Viannia) pifanoi, L. (Viannia) shawi, L. tarentolae, L. tropica, L. turanica* or *L. venezuelensis.*

The type 1 interferon receptor antagonist may be used as a single agent in treating leishmaniasis or in combination with an anti-kinetoplastid agent that is used to treat patients suffering from a leishmanial infection, such as sodium stibogluconate, meglumine antimonite, liposomal amphotericin B, miltefosine, pentamidine or antibiotics.

In certain embodiments, the type 1 interferon receptor antagonist may be used as a single agent in treating African trypanosomiasis, sleeping sickness or Chagas disease or in combination with an anti-kinetoplastid agent that is used to treat patients suffering from a trypanosomal infection, such as pentamidine, suramin, melarsoprol, eflornithine, or nifurtimox.

The infectious disease that can be is tularemia. In specific embodiments, the tularemia infection is caused by an agent selected from *Francisella tularensis, F. novicida, F. hispaniensis, F. persica comb. nov., F. noatunensis, F. philomiragia, F. halioticida, F. endociliophora, F. guangzhouensis* and *F. piscicida.*

The type 1 interferon receptor antagonist may be used as a single agent in treating tularemia or in combination with an antibiotic agent that is used to treat patients with a tularemia infection, such as streptomycin, gentamicin, amikacin, chloramphenicol, tetracycline, erythromycin or fluoroquinolones (ciprofloxacin, levofloxacin).

### Type 1 interferon receptor antagonists

The terms "Interferon type 1 receptor", "type 1 interferon receptor", "IFNAR" are used interchangeably, and include variants and isoforms. IFNAR is a heteromeric cell surface receptor composed of one chain with two subunits referred to as IFNAR1 and IFNAR2. The complete cDNA sequence of human IFNAR1 has the Genbank accession number NM_000629. The complete cDNA sequences of human IFNAR2 have the Genbank accession numbers (NM_207585, NM_000874, NM_001289125, NM_001289126 and NM_001289128).

Typically, the type 1 interferon receptor antagonist of the invention specifically binds to human IFNAR1 and prevents or inhibits human type 1 IFN signalling. In some embodiments, the type 1 interferon receptor antagonist of the invention, e.g. an antibodies that specifically binds to human IFNAR1 may cross-react with IFNARs from other species. More typically, however, the type 1 interferon receptor antagonist of the invention is specific for human IFNAR and does not exhibit cross-reactivity with IFNARs from other species. In some embodiments the type 1 interferon receptor antagonist of the invention specifically binds to an epitope on the human isoform of IFNAR1.

As used herein, the term "specific binding" refers to a binding reaction between the type 1 interferon receptor antagonist of the invention and IFNAR1 and/or IFNAR2 in which the dissociation constant (KD) is 10⁻⁷ M or less, in particular 10⁻⁸ M or less, 10⁻⁹ M or less or 10⁻¹⁰ M or less. The term "KD", as used herein, is intended to refer to the dissociation constant, which is obtained from the ratio of dissociation rate (Kd) to association rate (Ka) and is expressed as a molar concentration (M). KD values can be determined using methods well established in the art. One method for determining the association and disassociation dynamics of an antibody is by using surface plasmon resonance, e.g. by using a biosensor system such as a Biacore™ system.

In general, a smaller KD value (i.e. higher affinity) is preferred. Typically, for the type 1 interferon receptor antagonist to prevent or inhibit human type 1 IFN signalling, the affinity of the type 1 interferon receptor antagonist for human IFNAR1 and/or human IFNAR2 needs to be comparable to the affinity of type 1 human IFN to human IFNAR1 and/or human IFNAR2. In a specific embodiment, the KD of the binding reaction between the type 1 interferon receptor antagonist of the invention and IFNAR1 and/or IFNAR2 is 10⁻⁹ M or less, preferably 10⁻¹⁰ M or less.

Type 1 interferon receptor antagonists may act in various ways to inhibit signalling of type 1 IFNs. The type I IFN receptor relies on associated Janus kinases (JAKs) to phosphorylate receptors and signal transducing molecules, such as STAT proteins, after ligand-induced receptor clustering. IFNAR1 binds to a JAK named tyrosine kinase 2 (TYK2). IFNAR2 binds JAK1. The type 1 interferon receptor antagoni may interfere with binding of TYK2 and/or JAK1 to IFNAR1 and/or IFNAR2. For example, a type I IFN-inducible cysteine protease, UBP43, was shown to interact directly with IFNAR2, blocking the interaction between JAK1 and the receptor. Peptides derived from UBP43 may likewise block the interaction between JAK1 and the receptor. In other embodiments, the type 1 interferon receptor antagonist of the invention prevents ligand-induced association of IFNAR1 e.g. by steric hindrance. The interferon type 1 receptor antagonists of the invention prevents binding of type 1 IFNs to the type IFN receptor, e.g. by blocking access to the type 1 IFN binding side of IFNAR1 and/or IFNAR2.

The type 1 interferon receptor antagonist of the invention is an anti-type 1 interferon receptor antibody. The anti-type 1 interferon receptor antibody can be a human antibody, a chimeric antibody, a humanized antibody, or an antigen-binding fragment thereof. In a specific embodiment, the type 1 interferon receptor antagonist of the invention is a human anti-type 1 interferon receptor antibody or an antigen-binding fragment thereof. In some embodiments, an anti-type 1 interferon receptor antibody, or an antigen binding fragment thereof, is selected from the group consisting of IgG, F(ab')₂, F(ab)₂, Fab', Fab, ScFvs, diabodies, triabodies and tetrabodies.

The anti-type 1 interferon receptor antibody of the invention typically binds INFAR1 with high affinity. As used herein, the term "high affinity" refers to an antibody that binds INFAR1 with a KD of 10⁻⁷ M or less, 10⁻⁸ M or less, 10⁻⁹ M or less, or 10⁻¹⁰ M or less. However, "high affinity" binding can vary for different antibodies. For example, "high affinity" binding for an IgG antibody refers to a KD of 10⁻⁸ M or less, 10⁻⁹ M or less, or 10⁻¹⁰ M or less, whereas high affinity binding for an IgM antibody refers to an antibody having a KD of 10⁻⁷ M or less, or 10⁻⁸ M or less. In some embodiments, the anti-type 1 interferon receptor antagonist is an lgG1 or lgG4 antibody or antigen-binding fragment thereof.

In some embodiments, anti-type 1 interferon receptor antibodies for use in methods of the invention will bind to IFNAR1 with KD values in the range of 10⁻⁷ M to 10⁻¹¹ M and partially (by at least 50%), or substantially (by at least 70%) inhibits a known biological read-out of type 1 interferon signalling.

Inhibition of type 1 interferon signalling can be assessed in various ways. Biological read-outs that provide a useful correlate for the inhibition of type 1 interferon signalling include viral replication or cell proliferation assays [29]. For example, the ability of an anti-type 1 interferon receptor antibody of the invention to inhibit type 1 interferon signalling can be assessed by infecting A549 human lung carcinoma cells with encephalomyocarditis virus in the presence or absence of such an antibody. Viral replication is inhibited by type 1 interferon signalling. The extent of viral replication correlates with a cytopathic effect observed in infected cells. Alternatively, cell proliferation, e.g. of Daudi Burkitt's lymphoma cells, can be observed in the presence of type 1 interferon. If type 1 interferon signalling is effectively blocked by an anti-type 1 interferon receptor antibody, cell proliferation is reduced. Hence inhibition of type 1 interferon signalling can be assessed by comparing cell proliferation in the presence or absence of such an antibody.

Immunomodulatory assays of type I interferon activity can also be used to measure inhibition of type 1 interferon signalling [29]. Such assays detect the expression of type 1 interferon-inducible proteins. Change of expression of viral antigens in cells infected with a virus may also be measured. In some cases, the cells in which the designated protein is expressed are chemically fixed and the protein detected and quantified by binding of antibody specific for the protein or antigen of interest, followed by anti-antibody-enzyme conjugate and substrate conversion to a coloured metabolite. In other cases, the induced protein is extracted from the cells and quantified by an immunoassay specific for the protein. Suitable type 1 interferon-inducible proteins for use in this type of assay include cell surface proteins such as MHC class I and II protein and ICAM-1 and other cellular adhesion molecules. Where the protein of interest has enzymatic activity, it is possible to directly measure it. For example, 2-5A synthetase, PKR, RNase L and MxA GTPase are enzymes that may form the basis of such bioassays. An exemplary bioassay for IFN-α is based on induction and quantification of 2-5A synthetase. Alternatively, the type 1 interferon-inducible gene is a secreted protein or peptide such as the chemokine IP-10. Secreted proteins can be detected by standard methods such as ELISA. For example, inhibition of type 1 interferon signalling can be assessed by incubating peripheral blood mononuclear cells with a type 1 interferon in the presence or absence of an antibody of the invention.

Biological read-outs can also be obtained from so-called reporter gene assays. In such assays, the promoter of a known type 1 interferon-regulated gene (e.g. the Mx gene) is linked to a reporter gene encoding an easily detectable protein (e.g. a firefly luciferase or an alkaline phosphatase). Reporter cells stably transfected with such reporter gene constructs express the reporter gene product when exposed to type 1 interferon. Inhibition of type 1 interferon signalling can be assessed by incubating such cells in the presence or absence of a type 1 interferon antibody of the invention.

In certain embodiments, the type 1 interferon receptor antagonist inhibits the antiviral effects of type 1 IFNs with an IC₅₀ value of 0.001 to 5 µg/ml, e.g. 0.001 to 4 µg/ml, 0.001 to 2.5 µg/ml, 0.001 to 2 µg/ml, 0.001 to 1 µg/ml, 0.001 to 0.5 µg/ml, 0.01 to 5 µg/ml, 0.5 to 5 µg/ml, 0.01 to 2 µg/ml, 0.02 to 1 µg/ml, 0.02 to 0.5 µg/ml, or 0.02 to 0.05 µg/ml. The antiviral effects of IFNAR can be measured by the ability of the type 1 interferon receptor antagonist to neutralize the antiviral effects of human type 1 IFNs on WISH cells infected with VSV. In this assay, ED₅₀ doses of human IFN-alpha A and human IFN-beta (14.7 IU/ml and 5.03 IU/ml, respectively) can be used to test the effect of the type 1 interferon receptor antagonist [30].

In certain embodiments, the type 1 interferon receptor antagonist inhibits the antiproliferative effects of type 1 IFNs with an IC₅₀ value of 0.001 to 50 µg/ml, for example 0.001 to 40 µg/ml, 0.001 to 30 µg/ml, 0.001 to 20 µg/ml, 0.001 to 10 µg/ml, 0.001 to 5 µg/ml, 0.01 to 50 µg/ml, 0.2 to 30 µg/ml, 0.5 to 30 µg/ml, 0.01 to 20 µg/ml, 0.02 to 10 µg/ml, 0.02 to 5 µg/ml, 0.02 to 2 µg/ml. Dose-response experiments with human IFN-alpha A and human IFN-beta can be used to show inhibition of Daudi cell proliferation with an ED50 dose of 31 IU/ml and 49 IU/ml, respectively [30].

In certain embodiments, the type 1 interferon receptor antagonist inhibits the type 1 IFN-induced upregulation of MHC class I expression with an IC₅₀ value of 0.001 to 8 µg/ml, for example 0.001 to 5 µg/ml, 0.001 to 2.5 µg/ml, 0.001 to 2 µg/ml, 0.001 to 1 µg/ml, 0.001 to 0.5 µg/ml, 0.01 to 5 µg/ml, 0.5 to 5 µg/ml, 0.01 to 2 µg/ml, 0.02 to 1 µg/ml, 0.02 to 0.5 µg/ml, 0.02 to 0.05 µg/ml. Dose-response studies with human IFN-alpha A and human IFN-beta can be used to evaluate the MHC class I upregulation in human fibroblasts [30].

The level of inhibition of type 1 interferon signalling can also be measured by assessing the expression level or posttranslational modifications on one or more interferon stimulated genes (ISGs). In some embodiments, the level of inhibition of type 1 interferon signalling can be measured by detecting the expression levels of one or more gene products selected from the PKR (also known as EIF2AK2), MX1, OAS1, APOBEC3G, TRIM5, ZAP, ISG15, ADAR, IFITM1/2/3, tetherin (also known as BST2), and viperin (also known as RSAD2).

STAT and ISGF3-induced gene transcription is regulated at several levels, by posttranslational modification of STATs in the nucleus, through the cooperation of STATs with other transcription factors, by epigenetic factors and chromatin states at target gene loci that modulate the access of STATs to their target genes, through the interaction of STATs with co-activators, corepressors, chromatin-modifying complexes and transcription elongation factors, and by the binding of STATs to distal regulatory elements, such as enhancers. Tyrosine and serine phosphorylation of STATs occurs in the cytoplasm and serine phosphorylation of STATs also occurs in the nucleus, possibly at target gene loci. This nucleic phosphorylation event is mediated in part by cyclin- dependent kinase 8 (CDK8; which is a component of the Mediator co-activator complex) after STAT1 binds DNA. CDK8 modulates IFNγ-induced gene expression and augments antiviral responses, but its functional role in IFNAR signalling is not yet known. STATs are also acetylated, methylated and sumoylated. Therefore, in some embodiments the level of inhibition of type 1 interferon signalling can be measured by detecting the type and/or amount of posttranslational modification of STAT1, STAT2, JAK1 and/or TYK2 proteins in a cell treated with a type 1 interferon receptor antagonist of the invention. In a specific embodiment, the level of inhibition of type 1 interferon signalling can be measured by detecting the type and/or amount of posttranslational modification of STAT1 and/or STAT2 proteins in a cell treated with a type 1 interferon receptor antagonist. In another specific embodiment, the level of inhibition of type 1 interferon signalling can be measured by detecting the type and/or amount of posttranslational modification of JAK1 and/or TYK2 proteins in a cell treated with a type 1 interferon receptor antagonist. In some embodiments, the level of phosphorylation, acetylation, methylation and/or sumoylation of STAT1 can be used to measure the level of inhibition of type 1 interferon signalling. In other embodiments, the level of phosphorylation, acetylation, methylation and/or sumoylation of STAT2 can be used to measure the level of inhibition of type 1 interferon signalling. In one particular embodiment, the level of phosphorylation of STAT1 and/or STAT2 is detected to measure the level of inhibition of type 1 interferon signalling. In some embodiments, the level of phosphorylation, acetylation, methylation and/or sumoylation of JAK1 can be used to measure the level of inhibition of type 1 interferon signalling. In other embodiments, the level of phosphorylation, acetylation, methylation and/or sumoylation of TYK2 can be used to measure the level of inhibition of type 1 interferon signalling.

Methods of detecting protein expression levels and/or posttranslational modifications are well known to those skilled in the art. Exemplary techniques include Western blotting, mass spectrometry, Enzyme-Linked Immunosorbent Assay (ELISA) and flow cytometry (e.g. FACS).

### Type 1 interferons

The terms "interferon type 1", "type 1 interferon" and "type I interferon" are used interchangeably and intended to refer to, either collectively or separately, IFN-α (alpha), IFN-β (beta), IFN-ω (omega), IFN-κ (kappa) and IFN-ε (epsilon). In a particular embodiment, the term refers to IFN-α and IFN-β.

Type 1 IFNs are a gene family that consists of about 20 members, with some variation between mammalian species. Mammalian genomes contain IFN-α, IFN-β, IFN-ω, IFN-κ and IFN-ε genes. All type 1 IFNs share a variable degree of structural homology but no significant resemblance to IFN-γ. During viral and bacterial infections, the main type 1 IFNs that are synthesized are IFN-α and IFN-β [7]. A single IFN-β gene is found in most mammals, but the IFN-α family is formed by 13 functional genes in humans and 14 in mice. The human subtypes are IFNA1, IFNA2, IFNA4, IFNA5, IFNA6, IFNA7, IFNA8, IFNA10, IFNA13, IFNA14, IFNA16, IFNA17, IFNA21. The specific activity of individual IFN-α proteins differs. There are large differences in the ability of individual IFN-α proteins to activate natural killer (NK) cells. This is most striking in the case of human IFN-α7, which completely lacks this ability. The biological significance of having many IFN-α genes, in addition to an IFN-β gene, is not clear.

In some embodiments, the interferon type 1 receptor antagonists for use in methods of the invention inhibits signalling by one or more (e.g. all) of the human type 1 interferon subtypes, in particular human IFN-α and IFN-β. The coding sequences of the human type I IFNs are known. The complete cDNA sequence of human IFNA1 has the Genbank accession number NM_024013. The complete cDNA sequence of human IFNA2 has the Genbank accession number NM_000605. The complete cDNA sequence of human IFNA4 has the Genbank accession number NM_021068. The complete cDNA sequence of human IFNA5 has the Genbank accession number NM_002169. The complete cDNA sequence of human IFNA6 has the Genbank accession number NM_021002. The complete cDNA sequence of human IFNA7 has the Genbank accession number NM_021057. The complete cDNA sequence of human IFNA8 has the Genbank accession number NM_002170. The complete cDNA sequence of human IFNA10 has the Genbank accession number NM_002171. The complete cDNA sequence of human IFNA13 has the Genbank accession number NM_006900. The complete cDNA sequence of human IFNA14 has the Genbank accession number NM_002172. The complete cDNA sequence of human IFNA16 has the Genbank accession number NM_002173. The complete cDNA sequence of human IFNA17 has the Genbank accession number NM_021268. The complete cDNA sequence of human IFNA21 has the Genbank accession number NM_002175. The complete cDNA sequence of human IFNB1 has the Genbank accession number NM_002176. The complete cDNA sequence of human IFNK has the Genbank accession number NM_020124. The complete cDNA sequence of human IFNW1 has the Genbank accession number NM_002177.

Type 1 IFNs share a common cellular receptor (IFNAR), suggesting that their receptor-binding areas are highly conserved. Therefore, without being bound by any particular theory, the inventors believe that an interferon type 1 antagonist of the invention that typically prevents or inhibits the binding of one type 1 IFN to IFNAR1 prevents or inhibits the binding of all type 1 IFNs.

Three-dimensional models suggest that the globular structure of type 1 IFNs consists of a bundle of 5 alpha-helices, which may form two polypeptide domains. Disulphide bonds stabilize both domains in a bioactive configuration. Two conserved hydrophilic regions appear to constitute the basic framework of the receptor recognition site in type 1 IFNs. IFN-beta, IFN-omega and some IFN-alpha subtypes are glycoproteins, but the sugar moiety appears to be neither structurally nor functionally relevant.

### Anti-type 1 interferon receptor antibodies

The term "antibody" as used herein typically refers to both antibodies and antigen-binding fragments thereof. Antibodies include, but are not limited to, polyclonal, monoclonal, multispecific, mouse, human, humanized, primatized or chimeric antibodies. Typically, the anti-type 1 interferon receptor antibody of the invention, is a fully human, human or humanized antibody. An antigen-binding fragment, variant, or derivative of an anti-type 1 interferon receptor antibody of the invention includes, but is not limited to, a Fab, Fab' and F(ab')2, Fd, Fv, single-chain Fv (scFv), or disulfide-linked Fv (sdFv). ScFv molecules are known in the art and are described, in, for example, U.S. Patent No. 5,892,019 [31].

In some embodiments, an anti-type 1 interferon receptor antibody is IgG. In some embodiments, an anti-type 1 interferon receptor antibody is IgG1.

In some embodiments, a suitable anti-type 1 interferon receptor antibody contains an Fc domain or a portion thereof that binds to the FcRn receptor. As a non-limiting example, a suitable Fc domain may be derived from an immunoglobulin subclass such as IgG. In some embodiments, a suitable Fc domain is derived from IgG1, IgG2, IgG3, or IgG4. Particularly suitable Fc domains include those derived from human or humanized antibodies. It is contemplated that improved binding between Fc domain and the FcRn receptor results in prolonged serum half-life. Thus, in some embodiments, a suitable Fc domain comprises one or more amino acid mutations that lead to improved binding to FcRn. Various mutations within the Fc domain that effect improved binding to FcRn are known in the art and can be adapted to practice the present invention. In some embodiments, a suitable Fc domain comprises one or more mutations at one or more positions corresponding to Thr 250, Met 252, Ser 254, Thr 256, Thr 307, Glu 380, Met 428, His 433, and/or Asn 434 of human IgG1.

Methods for the generation and characterization of monoclonal mouse antibodies to the human interferon receptor have been described previously [3,32, 33]. Likewise, United States Patent US 7,662,381 B2 [34] teaches methods for generation of monoclonal human antibodies to human alpha interferon receptor 1 (IFNAR1).

In some embodiments, anti-type 1 interferon receptor antibodies for use in methods of the invention can be generated by immunising mice, preferably transgenic mice engineered to express human immunoglobulin genes, with preparations of soluble extracellular domains of IFNAR1 or IFNAR2, in the presence of suitable adjuvants such as Freund's adjuvant, obtaining B lymphocytes from spleen or lymph nodes of immunised mice, and developing hybridoma cells by fusion with myeloma cells using polyethylene glycol, a technique well known to those skilled in the art.

Hybridoma cells secreting antibodies to IFNAR1 or IFNAR2 can be identified by screening the culture supernatants for antibodies binding to isolated soluble extracellular domains of IFNAR1 or IFNAR2 or to cells expressing the full-length proteins, or by testing their ability to inhibit the biological activity of type 1 IFNs in cell culture experiments. The affinity of such antibodies for their target proteins can for example be determined using surface plasmon resonance technology (e.g. Biacore™). The antibodies raised against human IFNAR1 or IFNAR2 can then be selected for cross-reactivity with the corresponding proteins of non-human primates, such as rhesus monkeys (*Macacca mulatta*) or cynomolgus monkeys (*Macacca fascicularis*), a prerequisite for their use in non-human primate models of tuberculosis.

In some embodiments, the genes encoding the heavy and light chains of the antibody can be isolated from the selected hybridoma cells, engineered to alter the functional properties of the antibodies such as their affinity for Fc receptors, and cloned in expression vectors suitable for high-yield production of the antibody proteins in mammalian cells, such as Chinese hamster ovary (CHO) cells. Antibody proteins can then be isolated from the culture supernatants of these cells stably transfected with these genes, purified and formulated with excipients suitable for administration to humans.

In some embodiments, a suitable anti-type 1 interferon receptor antibody comprises complementarity determining regions (CDRs) with the sequences of:
i. SEQ ID NO: 1 for CDR1 of the heavy chain;
ii. SEQ ID NO: 2 for CDR2 of the heavy chain;
iii. SEQ ID NO: 3 for CDR3 of the heavy chain;
iv. SEQ ID NO: 4 for CDR1 of the light chain;
v. SEQ ID NO: 5 for CDR2 of the light chain; and
vi. SEQ ID NO: 6 for CDR3 of the light chain.

In some embodiments, a suitable anti-type 1 interferon receptor antibody comprises a heavy chain variable region of the amino acid sequence of SEQ ID NO: 7 and a light chain variable region of the amino acid sequence of SEQ ID NO: 8.

In some embodiments, a suitable anti-type 1 interferon receptor antibody comprises a heavy chain of the amino acid sequence of SEQ ID NO: 9 and a light chain of the amino acid sequence of SEQ ID NO: 10.

### Methods of treatment

Disclosed is a method of treating a subject suffering from an infectious disease in which type 1 IFN signalling is detrimental to the subject or promotes disease progression, said method comprising administering to the subject a therapeutically effective amount of a type 1 interferon receptor antagonist that specifically binds IFNAR1 and/or IFNAR2 and inhibits type 1 IFN signalling. Disclosed is the use of a type 1 interferon receptor antagonist that specifically binds IFNAR1 and/or IFNAR2 and inhibits type 1 IFN signalling in a method of treating a subject suffering from an infectious disease in which type 1 IFN signalling is detrimental to the subject or promotes disease progression.

As used herein, the terms "subject" or "individual" or "patient" refers to someone in need of therapy. As used herein, the term "subject" includes any human or nonhuman animal. The term "nonhuman animal" includes all vertebrates, e.g. mammals and non-mammals, such as mice, rats, nonhuman primates, sheep, dogs, cats, horses and cows. Typically, however, the term "subject" refers to a human.

The terms "effective amount" or "amount effective to" or "therapeutically effective amount" includes reference to a dosage of a therapeutic agent sufficient to produce a desired result, in particular the prevention of disease progression and/or the amelioration of symptoms associated with the disease for which the subject is being treated.

As used herein, the terms "treat", "treating" or "treatment" refer to therapeutic measures, wherein the object is to prevent or slow down (lessen) an undesired physiological change or disorder, such as the progression of an infectious disease. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total). "Treatment" can also mean prolonging survival as compared to expected survival if not receiving treatment. A subject in need of treatment typically refers to a patient who is already suffering from the disease, condition or disorder for which treatment is provided.

In some embodiments, the type 1 interferon receptor antagonist of the invention is administered in combination with one or more other therapeutic agent(s). For example, the combination therapy can include a type 1 interferon receptor antagonist of the invention combined with at least one other antimicrobial or antibacterial agent. For example, in a specific embodiment, the invention relates to a type 1 interferon receptor antagonist of the invention for use in treating tuberculosis in combination with one or more therapeutic agents selected from isoniazid, rifampicin, bedaquiline, delamanid, pyrazinamide or ethambutol. In another specific embodiment, the invention relates to the use of isoniazid, rifampicin, bedaquiline, delamanid, pyrazinamide or ethambutol in treating tuberculosis in combination with a type 1 interferon receptor antagonist of the invention.

Dosage regimens are typically adjusted to provide the optimum desired response (e.g. a therapeutic response). For example, a single bolus of the type 1 interferon receptor antagonist may be administered. In other embodiments, several divided doses may be administered over time or the dose may be proportionally reduced or increased as indicated by the needs of the therapeutic situation.

It is especially advantageous to formulate parenteral compositions in dosage unit form for ease of administration and uniformity of dosage. The term "dosage unit form" as used herein refers to physically discrete units suited as unitary dosages for the subjects to be treated; each unit contains a predetermined quantity of active compound calculated to produce the desired therapeutic effect in association with the required pharmaceutical carrier.

In some embodiments, a type 1 interferon receptor antagonist can be administered intravenously (IV), e.g. as an intravenous infusion or as an intravenous bolus. The term "intravenous infusion" refers to introduction of a drug, e.g. an anti-type 1 interferon receptor antibody or antigen-binding fragment thereof into the vein of an animal or human patient over a period of time greater than approximately 5 minutes, for example, between approximately 30 to 90 minutes, although, according to the disclosure, intravenous infusion is alternatively administered for 10 hours or less, e.g. 5 hours or less or 2 hours or less. In one particular embodiment, the duration of the infusion is at least 60 minutes. The term "intravenous bolus" or "intravenous push" refers to drug administration, e.g. of an anti-type 1 interferon receptor antagonist into a vein of an animal or human such that the body receives the drug in approximately 15 minutes or less, for example, 5 minutes or less.

In other embodiments, a type 1 interferon receptor antagonist of the invention can be administered subcutaneously. The term "subcutaneous administration" refers to introduction of the anti-type 1 interferon receptor antagonist under the skin of a subject, for example, within a pocket between the skin and underlying tissue, by relatively slow, sustained delivery from a drug receptacle. The pocket can be created by pinching or drawing the skin up and away from underlying tissue. In some embodiments, a composition comprising an type 1 interferon receptor antagonist is introduced under the surface of the skin of the patient with a hypodermic needle.

### Treatment and dosing using anti-type 1 interferon receptor antibodies

The invention relates to the use of an anti-type 1 interferon receptor antibody that specifically binds IFNAR1 and inhibits type 1 IFN signalling in a method of treating a subject suffering from an infectious disease in which type 1 IFN signalling is detrimental to the subject or promotes disease progression. Such a method comprises administering to the subject a therapeutically effective amount of the anti-type 1 interferon receptor antibody.

In some embodiments, the anti-type 1 interferon receptor antibody is administered in a dosage dependent on the subject's body weight. In certain embodiments, such weight-based dosage ranges from about 0.01 mg/kg to about 100 mg/kg of the subject's body weight. In certain embodiments, such weight-based dosage is chosen from about 0.3 mg/kg body weight, about 1 mg/kg body weight, about 3 mg/kg body weight, and about 10 mg/kg body weight.

In some embodiments, the antibody is administered as a single dose or is administered in two or more doses once per week, once every two weeks, once every three weeks, once every four weeks, once a month, once every 3 months, once every six months, or at varying intervals.

In certain embodiments, the administration is by a route chosen from intravenous, intramuscular, intraperitoneal, intracerobrospinal, subcutaneous, intra-articular, intrasynovial, intrathecal, oral, topical, inhalation, and a combination of two or more recited routes. In a specific embodiment, the anti-type 1 interferon receptor antibody is administered parenterally.

Typically, the anti-type 1 interferon receptor antibody of the invention is administered intravenously. In a specific embodiment, patients with an infectious disease can be treated with an anti-type 1 interferon receptor antibody of the invention by intravenous infusion at doses of 1 mg/kg to 100 mg/kg in intervals of 1 week to 4 weeks.

In some embodiments, the anti-type 1 interferon receptor antibodies can be given as a single agent, but preferably they are administered in combination with established chemotherapeutic agents commonly used for treatment of the infectious disease. For example, in some embodiments, the anti-type 1 interferon receptor antibodies can be given in combination with established chemotherapeutic agents commonly used for treatment of the tuberculosis patients, such as isoniazid, rifampicin, bedaquiline, delamanid, pyrazinamide or ethambutol.

### Pharmaceutical Compositions

The present disclosure also provides a composition, e.g. a pharmaceutical composition, comprising a type 1 interferon receptor antagonists of the invention. Such compositions may include one or a combination of (e. g. two or more different) type 1 interferon receptor antagonists of the invention. For example, a pharmaceutical composition of the invention can comprise two or more anti-type 1 interferon receptor antibodies that bind to different epitopes on the type 1 interferon receptor or that have otherwise complementary activities.

In some embodiments, the disclosure provides a pharmaceutical composition comprising a type 1 interferon receptor antagonists of the invention and a pharmaceutically acceptable carrier. The term "pharmaceutically acceptable carrier" includes any and all solvents, buffers, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e. g. by injection or infusion). For example, in some embodiments, a composition for intravenous administration typically is a solution in sterile isotonic aqueous buffer.

Depending on the route of administration, the type 1 interferon receptor antagonists according to the invention, may be coated in a material to protect the compound from the action of acids and other natural conditions that may inactivate the compound. For example, oral formulations can include standard carriers such as pharmaceutical grades of mannitol, lactose, starch, magnesium stearate, polyvinyl pyrrolidone, sodium saccharine, cellulose, magnesium carbonate etc.

In some embodiments, a pharmaceutical composition of the invention comprises an anti-type 1 interferon receptor antibody or antigen-binding fragment thereof in form of an injectable formulation. In other embodiments, a pharmaceutical composition of the inventions comprises an anti-type 1 interferon receptor antibody or antigen-binding fragment thereof is formulated for parenteral administration, e.g. formulated for intravenous, subcutaneous, or intramuscular administration. Examples of pharmaceutical compositions comprising anti-type 1 interferon receptor antibodies can be found in PCT Application Publication WO2013188494 [35] and PCT Application Publication WO2006002177 [36].

In some embodiments, pharmaceutically acceptable carriers include sterile aqueous solutions or dispersions and sterile powders for the extemporaneous preparation of sterile injectable solutions or dispersion. In certain embodiments, the disclosure provides a sterile powder of the type 1 interferon receptor antagonist for the preparation of sterile injectable solutions. In the case of sterile powders for the preparation of sterile injectable solutions, the preferred methods of preparation are vacuum drying and freeze-drying (lyophilization) that yield a powder of the type 1 interferon receptor antagonist plus any additional desired ingredient from a previously sterile-filtered solution thereof. Where the composition is to be administered by infusion, it can be dispensed with an infusion bottle containing sterile pharmaceutical grade water, saline or dextrose/water. Where the composition is administered by injection, an ampule of sterile water for injection or saline can be provided so that the ingredients may be mixed prior to administration.

### General

The term "comprising" encompasses "including" as well as "consisting", e.g. a composition "comprising" X may consist exclusively of X or may include something additional, e.g. X + Y.

The term "about" or "approximately" in relation to a numerical value x means, for example, x+10%.

### EXAMPLES

### Example 1: Efficacy of antibodies to IFNAR1 in a mouse model of tuberculosis

A total of 40 mice (strain 129SvPas) were infected with *M*. *tuberculosis* strain H37Rv using a standard low-dose aerosol (LDA) infection with 100 colony forming units (CFUs). The infected mice were checked daily for clinical signs of disease and their body weight was monitored at least twice per week.

Three weeks after infection the mice in the treatment group were treated with an intraperitoneal injection of mouse anti-mouse IFNAR1 antibody (clone MAR1-5A3; Leeinco Technologies, Inc. USA), which was administered twice per week at a dose of 20 mg/kg for the remainder of the experiment. Mice in the control group were treated with PBS.

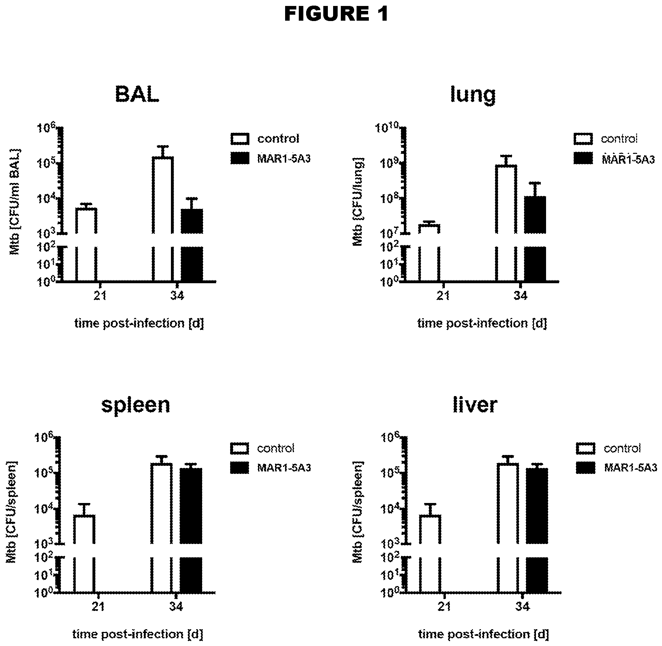

At 21 and 34 days post infection, 5 mice from each group were sacrificed and their lungs, liver, spleen and kidneys were collected. Serum was collected for analysis by terminal phlebotomy. Prior to isolation of the lungs, bronchoalveolar lavage (BAL) was performed with 1 ml of phosphate-buffered saline (PBS). The wash was plated to determine the number of CFUs. Hemi-sections were prepared from the isolated lung, liver and spleen materials. One part of the hemisected lung, liver and spleen materials was homogenized. A sample from each homogenate was plated to determine CFU counts. The CFU counts observed in each of the spleen, lung, liver and BAL fractions for the control and treated samples are shown in Figure 1.

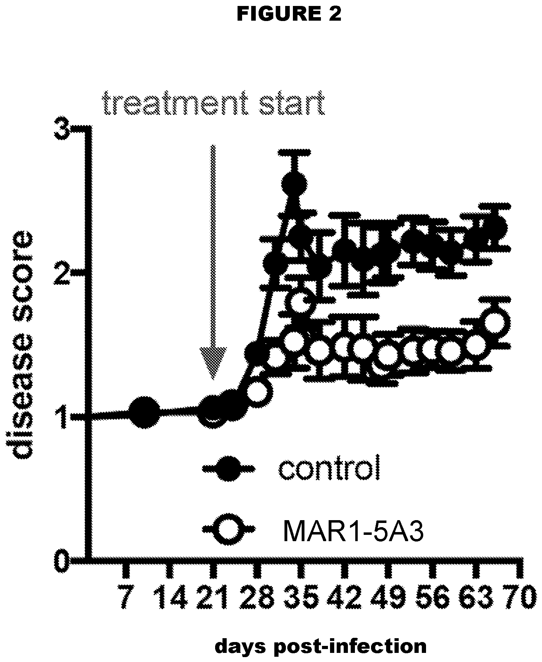

Additionally, mice were also monitored every week - or twice or five times per week when an average disease score of 2.5 or 3.0, respectively, was reached, up to a total of 77 days post-infection. For each of these time points a disease score was calculated by observation of a combination of factors, including overall activity, change of body weight, general condition and spontaneous behaviour. The disease score calculation is shown in Table 1. This was done for both the control group and the mice treated with the MAR1-5A3 antibody. The results are summarised in Figure 2.

Histopathology was performed on fixed sections of the lungs, spleen and kidneys. Tissue was embedded into a single tissue block ready for staining with hematoxylin and eosin stain (H&E). Mycobacteria can be visualised with Ziehl-Neelson stain (or an equivalent acid fast red stain).

Mice treated with the anti-mouse IFNAR1 antibody showed less weight loss than control mice, and a reduction in the number of mycobacteria was observed relative to the control mice. The results demonstrate the efficacy of treating tuberculosis with an anti-type 1 interferon receptor antibody.

**Table 1 - Disease score calculation**

| **Disease Score** | **Activity** | **Body Weight** | **General Condition** | **Spontaneous Behaviour** |
|---|---|---|---|---|
| **1** | Very active | Unaffected or increased | Coat smooth, shiny; Orifices clean; eyes clear, bright | normal behaviour |
| **2** | active | Change <10 % | Coat imperfections (reduced or increased body care) | Minimal deviations from normal behaviour |
| **3** | less active | Weight reduction 10-20 % | Coat dull, disordered, unkempt orifices; increased muscle tonus | Unusual behaviour, restricted motor activity or hyperkinetic |
| **4** | merely active | Weight reduction 20-30 % | Dirty coat, clotted or watery orifices, abnormal posture, eyes turbid; high muscle tonus | Self-isolation, lethargy; pronounced hyperkinetic, stereotypes of behaviour, coordination disorders |
| **5** | lethargic | Weight reduction >30% | Cramping, paralysis (musculature of trunk, extremities); respiratory sounds; cold skin/body | Signs of pain upon touching, self-mutilation (auto aggression) |

### Example 2 - Efficacy of antibodies to IFNAR1 in a monkey model of tuberculosis

To determine the efficacy of therapy with an anti-type 1 interferon receptor antibody in treating tuberculosis in a model closer to humans, rhesus monkeys were used as a non-human primate model. The animals selected for the study were preferably of the same sex, between 4 to 8 years of age and had a body weight between 5 and 12 kg. The animals were subjected to a health check and were screened for antimycobacterial immunity, specific viruses, pathogenic ecto- and endoparasites and common bacteriological infections.

Two weeks before infection, blood and serum samples were collected and CT or PET/CT images of the lungs were taken to determine a pre-infection baseline for each animal. All animals received a low dose infectious challenge with *M. tuberculosis* strain Erdman K01. Animals were anesthetized with ketamine (10 mg/kg) followed by an injection of atropine (0.04 mg/kg). Lidocaine (0.1%) was sprayed onto the epiglottis prior to intubation. The mycobacteria were endobronchially delivered to the right lower lung lobe through a flexible fiber-optic bronchoscope at a targeted dose of 15 colony-forming units (CFU) in a volume of 3 mL.

Therapeutic intervention with a monoclonal antibody to the type 1 interferon receptor started 7 days post infection. It is continued at a frequency of once every 2 weeks, for a total of 6 administrations. For each administration, a dose of 30 mg antibody per kg body weight is infused intravenously over a period of about 15 minutes. Blood sampling is performed once every 2 weeks, just prior to antibody administration. At the time of the last antibody administration, a last blood sample is taken. Subsequently the animals are euthanized as per fixed endpoint of the study.

Blood is collected from the femoral vein under sedation using Vacutainers. Clotted blood is centrifuged. The serum is harvested and one part is used for determination of clinical chemistry (see below). Heparinized blood is used for the isolation of peripheral blood mononuclear cells (PBMC), which may be used in a variety of assays immediately and/or stored frozen. Ethylenediaminetetraacetic acid (EDTA) blood is used for determination of haematological parameters. Separate blood samples are taken for extraction of RNA, using PAXgene blood RNA tubes. RNA is extracted to determine changes in the IFN gene signature as a pharmacodynamics marker of IFNAR1 blockade [37].

Daily observation and examination for outward signs of disease (such as possible apathy, labour of breathing and (loss of) appetite) is performed. Body weight is recorded each time the animals are sedated. Positron Emission Tomography/Computed Tomography (PET/CT) imaging is applied to visualize granuloma formation/resorption and host responses in the lungs.

Animals are euthanized because of anti-drug antibodies (ADA) formation in the antibody-treated animal or because of humane endpoint or at the endpoint fixed and will undergo a full necropsy procedure. In the case of euthanasia of an animal due to ADA formation or because of humane endpoint, the paired animal will also be euthanized. As part of the assessments, macroscopic manifestation of tuberculosis will be scored by a standardized protocol. Beside gross pathology evaluation, tissue samples are collected for histopathology and for bacteriology as well as for local immune response measurements. Mycobacterial load is measured in homogenates of lungs, spleen and bronchoalveolar lymph nodes that are serially diluted and plated on culture medium for enumeration of CFUs.

Animals treated with the anti-type 1 interferon receptor antibody show less weight loss, lung inflammation and lung necrosis and a reduction in the number of mycobacteria relative to untreated control animals. Importantly, treated animals survive longer than controls. The results demonstrate the efficacy of treating tuberculosis with an anti-type 1 interferon receptor antibody.

### REFERENCES

- [1]: Cutrone and Langer (2001) J. Biol. Chem. 276:17140
- [2]: Uze et al. (1990) Cell 60:225
- [3]: Novick et al. (1994) Cell 77:391
- [4]: Cook et al. (1996) J. Biol. Chem. 271:13448
- [5]: Lewerenz et al. (1998) J. Mol. Biol. 282: 585
- [6]: Stark et al. (1998) Annu Rev Biochem; 67:227-64
- [7]: Haller et al. (1981) J. Exp. Med. 154:199
- [8]: Brinkmann et al. (1993) J. Exp. Med. 178:1655
- [9]: Finkelman et al. (1991) J. Exp. Med. 174:1179
- [10]: Santini et al. (2000) J. Exp. Med. 191:1777
- [11]: Furie et al., (2016) Arthritis & Rheumatology DOI 10.1002/art.39962
- [12]: Manca et al. (2005) J. Interferon Cytokine Res. 25, 694-701.
- [13]: Ordway et al. (2007) J. Immunol. 179, 522-531.
- [14]: Stanley et al. (2007) J. Immunol. 178, 3143-3152.
- [15]: Manca et al. (2001) Proc. Natl Acad. Sci. USA 98, 5752-5757.
- [16]: Cooper et al. (2000) Infect. Immun. 68, 6879-6882.
- [17]: Berry et al. (2010) Nature 466, 973-977.
- [18]: Cliff et al. (2013) J. Infect. Dis. 207, 18-29.
- [19]: Maertzdorf et al. (2011) Genes Immun. 12, 15-22.
- [20]: Ottenhoff et al. (2012) PLoS ONE 7, e45839.
- [21]: Antonelli et al. (2010) J. Clin. Invest. 120, 1674-1682.
- [22]: Mayer-Barber et al. (2011) Immunity 35, 1023-1034.
- [23]: McNab et al. (2013) J. Immunol. 191, 1732-1743.
- [24]: Redford et al. (2014) J. Infect. Dis. 209, 270-274.
- [25]: Mayer-Barber et al. (2010) J. Immunol. 184, 3326-3330.
- [26]: de Paus et al. (2013) Cytokine 61, 645-655.
- [27]: Novikov et al. (2011) J. Immunol. 187, 2540-2547.
- [28]: McNab et al. (2014) J. Immunol. 193, 3600-3612.
- [29]: Meager (2002) J. Immunol. Methods 261, 21-36
- [30]: Novick et al. (2000) J. Interferon & Cytokine Research 20:971-982
- [31]: US 5,892,019
- [32]: Goldman et al. (1999) J Interferon Cytokine Res 19:15
- [33]: Benoit et al. (1993) J Immunol 150:707
- [34]: US 7,662,381
- [35]: WO2013188494
- [36]: WO2006002177
- [37]: Goldberg et al. (2014) Arthritis Research and Therapy 16:R57

## Claims

1. A type 1 interferon receptor antagonist for use in a method of treating tuberculosis in a subject in which type 1 IFN signalling is detrimental to the subject or promotes disease progression, wherein the type 1 interferon receptor antagonist: (i) specifically binds IFNAR1 and inhibits signalling of type 1 IFNs; and (ii) is an anti-type 1 interferon receptor antibody or an antigen-binding fragment thereof.

2. The type 1 interferon receptor antagonist for use according to claim 1, wherein the anti-type 1 interferon receptor antibody is a polyclonal, monoclonal, multispecific, mouse, human, humanized, primatized, a chimeric antibody or a single-chain antibody.

3. The type 1 interferon receptor antagonist for use according to claim 1 or claim 2, wherein the anti-type 1 interferon receptor antibody or antigen-binding fragment thereof is selected from a Fab, Fab', F(ab')2, Fd, Fv, a single-chain Fv (scFv) and a disulfide-linked Fvs (sdFv), optionally wherein the anti-type 1 interferon receptor antibody is human or humanized.

4. The type 1 interferon receptor antagonist for use according to claim 3, wherein the anti-type 1 interferon receptor antibody is a monoclonal antibody.

5. The type 1 interferon receptor antagonist for use according to any one of claims 1 to 4, wherein the antibody or antigen-binding fragment, comprises complementarity determining regions (CDRs) with the sequences of:
a. SEQ ID NO: 1 for CDR1 of the heavy chain;
b. SEQ ID NO: 2 for CDR2 of the heavy chain;
c. SEQ ID NO: 3 for CDR3 of the heavy chain;
d. SEQ ID NO: 4 for CDR1 of the light chain;
e. SEQ ID NO: 5 for CDR2 of the light chain; and
f. SEQ ID NO: 6 for CDR3 of the light chain.
optionally wherein the antibody or antigen-binding fragment comprises a heavy chain variable region of the amino acid sequence of SEQ ID NO: 7 and a light chain variable region of the amino acid sequence of SEQ ID NO: 8, optionally wherein the antibody comprises a heavy chain of the amino acid sequence of SEQ ID NO: 9 and a light chain of the amino acid sequence of SEQ ID NO: 10.

6. The type 1 interferon receptor antagonist for use according to any of claims 1 to 5, wherein the tuberculosis:
(a) is caused by an agent selected from: *Mycobacterium tuberculosis, M. africanum, M. bovis, M. bovis BCG, M. canetti, M. caprae, M. microti, M. mungi, M. orygis, M. pinnipedii, M. suricattae, M*. *kansasii, M. xenopi, M. scrofulaceum, M*. *abscessus, M. haemophilum* and *M. ulcerans;* and/or
(b) is drug-resistant tuberculosis, optionally wherein (i) the drug-resistant tuberculosis is resistant to at least one of isoniazid, rifampicin, bedaquiline, delamanid, pyrazinamide and ethambutol; or (ii) the tuberculosis is multidrug-resistant tuberculosis and is resistant to at least one of isoniazid or rifampicin and at least one other anti-mycobacterial agent.

7. The type 1 interferon receptor antagonist for use according to any preceding claim, wherein the type 1 interferon receptor antagonist is administered by intravenous, intramuscular, intraperitoneal, intracerobrospinal, subcutaneous, intra-articular, intrasynovial, intrathecal, oral or topical administration or by inhalation, optionally wherein the type 1 interferon receptor antagonist is administered by intravenous or subcutaneous administration.

8. The type 1 interferon receptor antagonist for use according to any preceding claim, wherein the type 1 interferon receptor antagonist is administered at a dosage of about 0.01 mg/kg to about 100 mg/kg of the subject's body weight, optionally wherein the type 1 interferon receptor antagonist is administered at a dosage of about 10 mg/kg to about 30 mg/kg of the subject's body weight.

9. The type 1 interferon receptor antagonist for use according to claim 8, wherein the type 1 interferon receptor antagonist is administered at a dosage of:
a. about 0.3 mg/kg body weight;
b. about 1 mg/kg body weight;
c. about 3 mg/kg body weight; or
d. about 10 mg/kg body weight.

10. The type 1 interferon receptor antagonist for use according to claim 8 or claim 9, wherein the type 1 interferon receptor antagonist is administered at a dosage of:
a. about 10 mg/kg body weight;
b. about 15 mg/kg body weight;
c. about 20 mg/kg body weight;
d. about 25 mg/kg body weight; or
e. about 30 mg/kg body weight.

11. The type 1 interferon receptor antagonist for use according to any preceding claim, wherein the type 1 interferon receptor antagonist is administered at a fixed dosage from about 50 mg to about 2000 mg, optionally wherein the type 1 interferon receptor antagonist is administered at a dosage of:
a. about 100 mg;
b. about 200 mg;
c. about 300 mg;
d. about 600 mg;
e. about 1000 mg;
f. about 1200 mg;
g. about 1500 mg; or
h. about 2000 mg.

12. The type 1 interferon receptor antagonist for use according to any preceding claim, wherein the type 1 interferon receptor antagonist is administered:
a. as a single dose;
b. in two or more doses once per week;
c. once every two weeks;
d. once every three weeks;
e. once every four weeks;
f. once a month;
g. once every 3 months; or
h. once every six months.

13. The type 1 interferon receptor antagonist for use according to any preceding claim, wherein the type 1 interferon receptor antagonist is administered in intervals of one day to six months, optionally wherein the type 1 interferon receptor antagonist is administered in intervals of:
a. 1 week;
b. 2 weeks;
c. 3 weeks;
d. 4 weeks;
e. 1 month;
f. 2 months;
g. 3 months;
h. 4 months;
i. 5 months; or
j. 6 months.

14. The type 1 interferon receptor antagonist for use according to any of preceding claim, wherein the type 1 interferon receptor antagonist is administered intravenously or subcutaneously at doses of 1 mg/kg to 30 mg/kg of bodyweight, at intervals of 1 week to 4 weeks.

15. The type 1 interferon receptor antagonist for use according to any preceding claim, said method comprising further administering to the subject a therapeutically effective amount of an additional therapeutic agent, optionally wherein the additional therapeutic agent is:
(a) an antimicrobial or antibacterial agent, optionally wherein the antimicrobial or antibacterial agent is selected from the list: isoniazid, rifampicin, bedaquiline, delamanid, pyrazinamide and ethambutol;
(b) an anti-kinetoplastid agent selected from sodium stibogluconate, meglumine antimonite, liposomal amphotericin B, miltefosine, pentamidine or antibiotics;
(c) an anti-kinetoplastid agent selected from pentamidine, suramin, melarsoprol, eflornithine, or nifurtimox; or
(d) an antibiotic agent selected from streptomycin, gentamicin, amikacin, chloramphenicol, tetracycline erythromycin or fluoroquinolones.

## Patentansprüche

1. Typ-1-Interferonrezeptorantagonist zur Verwendung in einem Verfahren zum Behandeln von Tuberkulose bei einem Subjekt, wobei die Typ-1-IFN-Signalisierung nachteilig für das Subjekt ist oder den Krankheitsverlauf fördert, wobei der Typ-1-Interferonrezeptorantagonist: (i) spezifisch IFNAR1 bindet und die Signalisierung von Typ-1-IFNs hemmt; und (ii) ein Anti-Typ-1-Interferonrezeptorantikörper oder ein antigenbindendes Fragment davon ist.

2. Typ-1-Interferonrezeptorantagonist zur Verwendung nach Anspruch 1, wobei der Anti-Typ-1-Interferonrezeptorantikörper ein polyklonaler, monoklonaler, multispezifischer, Maus-, Mensch-, humanisierter, primatisierter, ein chimärer Antikörper oder ein einkettiger Antikörper ist.

3. Typ-1-Interferonrezeptorantagonist zur Verwendung nach Anspruch 1 oder Anspruch 2, wobei der Anti-Typ-1-Interferonrezeptorantikörper oder das antigenbindende Fragment davon ausgewählt ist aus einem Fab, Fab', F(ab')2, Fd, Fv, einem einkettigen Fv (scFv) und einem disulfidgebundenen Fvs(sdFv), optional wobei der Anti-Typ-1-Interferonrezeptorantikörper menschlich oder humanisiert ist.

4. Typ-1-Interferonrezeptorantagonist zur Verwendung nach Anspruch 3, wobei der Anti-Typ-1-Interferonrezeptorantikörper ein monoklonaler Antikörper ist.

5. Typ-1-Interferonrezeptorantagonist zur Verwendung nach einem der Ansprüche 1 bis 4, wobei der Antikörper oder das antigenbindende Fragment Komplementarität bestimmende Region (CDRs) umfasst mit den Sequenzen:
a. SEQ ID NO: 1 für CDR1 der Schwerkette;
b. SEQ ID NO: 2 für CDR2 der Schwerkette;
c. SEQ ID NO: 3 für CDR3 der Schwerkette;
d. SEQ ID NO: 4 für CDR1 der Leichtkette;
e. SEQ ID NO: 5 für CDR2 der Leichtkette; und
f. SEQ ID NO: 6 für CDR3 der Leichtkette;
optional wobei der Antikörper oder das antigenbindende Fragment eine variable Region der Schwerkette der Aminosäuresequenz von SEQ ID NO: 7 und eine variable Region der Leichtkette der Aminosäuresequenz von SEQ ID NO: 8 umfasst, optional wobei der Antikörper eine Schwerkette der Aminosäuresequenz von SEQ ID NO: 9 und eine Leichtkette der Aminosäuresequenz von SEQ ID NO: 10 umfasst.

6. Typ-1-Interferonrezeptorantagonist zur Verwendung nach einem der Ansprüche 1 bis 5, wobei die Tuberkulose:
(a) verursacht wird durch einen Erreger, ausgewählt aus: *Mycobacterium tuberculosis, M. africanum, M. bovis, M. bovis BCG, M. canetti, M. caprae, M. microti, M. mungi, M. orygis, M. pinnipedii, M. suricattae, M. kansasii, M. xenopi, M. scrofulaceum, M. abscessus, M. haemophilum* und *M. ulcerans;* und/oder
(b) arzneimittelresistente Tuberkulose ist, optional wobei (i) die arzneimittelresistente Tuberkulose resistent ist gegen mindestens eines von Isoniazid, Rifampicin, Bedaquilin, Delamanid, Pyrazinamid und Ethambutol; oder (ii) die Tuberkulose eine multiresistente Tuberkulose ist und resistent ist gegen mindestens eines von Isoniazid oder Rifampicin und mindestens ein anderes antimykobakterielles Mittel.

7. Typ-1-Interferonrezeptorantagonist zur Verwendung nach einem der vorhergehenden Ansprüche, wobei der Typ-1-Interferonrezeptorantagonist verabreicht wird durch intravenöse, intramuskuläre, intraperitoneale, intrazerebrospinale, subkutane, intraartikuläre, intrasynoviale, intrathekale, orale oder topische Verabreichung oder durch Inhalation, optional wobei der Typ 1-Interferonrezeptorantagonist durch intravenöse oder subkutane Verabreichung verabreicht wird.

8. Typ-1-Interferonrezeptorantagonist zur Verwendung nach einem der vorhergehenden Ansprüche, wobei der Typ-1-Interferonrezeptorantagonist in einer Dosierung von etwa 0,01 mg/kg bis etwa 100 mg/kg des Körpergewichts des Subjekts verabreicht wird, optional wobei der Typ-1-Interferonrezeptorantagonist in einer Dosierung von etwa 10 mg/kg bis etwa 30 mg/kg des Körpergewichts des Subjekts verabreicht wird.

9. Typ-1-Interferonrezeptorantagonist zur Verwendung nach Anspruch 8, wobei der Typ-1-Interferonrezeptorantagonist in einer Dosis verabreicht wird von:
a. etwa 0,3 mg/kg Körpergewicht;
b. etwa 1 mg/kg Körpergewicht;
c. etwa 3 mg/kg Körpergewicht; oder
d. etwa 10 mg/kg Körpergewicht.

10. Typ-1-Interferonrezeptorantagonist zur Verwendung nach Anspruch 8 oder Anspruch 9, wobei der Typ-1-Interferonrezeptorantagonist in einer Dosis verabreicht wird von:
a. etwa 10 mg/kg Körpergewicht;
b. etwa 15 mg/kg Körpergewicht;
c. etwa 20 mg/kg Körpergewicht;
d. etwa 25 mg/kg Körpergewicht; oder
e. etwa 30 mg/kg Körpergewicht.

11. Typ-1-Interferonrezeptorantagonist zur Verwendung nach einem der vorhergehenden Ansprüche, wobei der Typ-1-Interferonrezeptorantagonist in einer festen Dosierung von etwa 50 mg bis etwa 2000 mg verabreicht wird, optional wobei der Typ-1-Interferonrezeptorantagonist verabreicht wird in einer Dosierung von:
a. etwa 100 mg;
b. etwa 200 mg;
c. etwa 300 mg;
d. etwa 600 mg;
e. etwa 1000 mg;
f. etwa 1200 mg;
g. etwa 1500 mg; oder
h. etwa 2000 mg.

12. Typ-1-Interferonrezeptorantagonist zur Verwendung nach einem der vorhergehenden Ansprüche, wobei der Typ-1-Interferonrezeptorantagonist verabreicht wird:
a. als Einzeldosis;
b. in zwei oder mehr Dosen einmal pro Woche;
c. einmal alle zwei Wochen;
d. einmal alle drei Wochen;
e. einmal alle vier Wochen;
f. einmal im Monat;
g. einmal alle 3 Monate; oder
h. einmal alle sechs Monate.

13. Typ-1-InterferonrRezeptorantagonist zur Verwendung nach einem der vorhergehenden Ansprüchen, wobei der Typ-1-Interferonrezeptorantagonist in Intervallen von einem Tag bis zu sechs Monaten verabreicht wird, optional wobei der Typ-1-Interferonrezeptorantagonist in Intervallen verabreicht wird von:
a. 1 Woche;
b. 2 Wochen;
c. 3 Wochen;
d. 4 Wochen;
e. 1 Monat;
f. 2 Monaten;
g. 3 Monaten;
h. 4 Monaten;
i. 5 Monaten; oder
j. 6 Monaten.

14. Typ-1-Interferonrezeptorantagonist zur Verwendung nach einem der vorhergehenden Ansprüche, wobei der Typ-1-Interferonrezeptorantagonist intravenös oder subkutan in einer Dosis von 1 mg/kg bis 30 mg/kg Körpergewicht in Intervallen von 1 Woche bis 4 Wochen verabreicht wird.

15. Typ-1-Interferon-Rezeptorantagonist zur Verwendung nach einem der vorhergehenden Ansprüche, wobei das Verfahren ferner das Verabreichen einer therapeutisch wirksamen Menge eines zusätzlichen Wirkstoffs umfasst, optional wobei der zusätzliche Wirkstoff Folgendes ist:
(a) ein antimikrobielles oder antibakterielles Mittel, optional wobei das antimikrobielle oder antibakterielle Mittel ausgewählt ist aus der Liste: Isoniazid, Rifampicin, Bedaquilin, Delamanid, Pyrazinamid und Ethambutol;
(b) ein Antikinetoplastidmittel, ausgewählt aus Natriumstibogluconat, Megluminantimonit, liposomalem Amphotericin B, Miltefosin, Pentamidin oder Antibiotika;
(c) ein Antikinetoplastidmittel, ausgewählt aus Pentamidin, Suramin, Melarsoprol, Eflornithin oder Nifurtimox; oder
(d) ein Antibiotikum, ausgewählt aus Streptomycin, Gentamicin, Amikacin, Chloramphenicol, Tetracyclin-Erythromycin oder Fluorchinolonen.

## Revendications

1. Antagoniste du récepteur de l'interféron de type 1 pour une utilisation dans un procédé de traitement de la tuberculose chez un sujet chez lequel la signalisation de l'IFN de type 1 est préjudiciable au sujet ou promeut une progression de la maladie, l'antagoniste du récepteur de l'interféron de type 1 : (i) se liant spécifiquement à l'IFNAR1 et inhibant la signalisation des IFN de type 1 ; et (ii) étant un anticorps anti-récepteur de l'interféron de type 1 ou un fragment de liaison à l'antigène de celui-ci.

2. Antagoniste du récepteur de l'interféron de type 1 pour une utilisation selon la revendication 1, l'anticorps anti-récepteur de l'interféron de type 1 étant un anticorps polyclonal, monoclonal, multispécifique, de souris, humain, humanisé, primatisé, un anticorps chimérique ou un anticorps monocaténaire.

3. Antagoniste du récepteur de l'interféron de type 1 pour une utilisation selon la revendication 1 ou la revendication 2, l'anticorps anti-récepteur de l'interféron de type 1 ou le fragment de liaison à l'antigène de celui-ci étant choisi parmi un Fab, un Fab', un F(ab')2, un Fd, un Fv, un Fv monocaténaire (scFv) et des Fv à liaison disulfure (sdFv), en option l'anticorps anti-récepteur de l'interféron de type 1 étant humain ou humanisé.

4. Antagoniste du récepteur de l'interféron de type 1 pour une utilisation selon la revendication 3, l'anticorps anti-récepteur de l'interféron de type 1 étant un anticorps monoclonal.

5. Antagoniste du récepteur de l'interféron de type 1 pour une utilisation selon l'une quelconque des revendications 1 à 4, dans lequel l'anticorps ou le fragment de liaison à l'antigène comprend des régions déterminant la complémentarité (CDR) ayant les séquences suivantes :
a. SEQ ID NO: 1 pour la CDR1 de la chaîne lourde ;
b. SEQ ID NO: 2 pour la CDR2 de la chaîne lourde ;
c. SEQ ID NO: 3 pour la CDR3 de la chaîne lourde ;
d. SEQ ID NO: 4 pour la CDR1 de la chaîne légère ;
e. SEQ ID NO: 5 pour la CDR2 de la chaîne légère ; et
f. SEQ ID NO: 6 pour la CDR3 de la chaîne légère,
éventuellement dans lequel l'anticorps ou le fragment de liaison à l'antigène comprend une région variable de chaîne lourde de la séquence d'acides aminés de SEQ ID NO: 7 et une région variable de chaîne légère de la séquence d'acides aminés de SEQ ID NO: 8, éventuellement dans lequel l'anticorps comprend une chaîne lourde de la séquence d'acides aminés de SEQ ID NO: 9 et une chaîne légère de la séquence d'acides aminés de SEQ ID NO: 10.

6. Antagoniste du récepteur de l'interféron de type 1 pour une utilisation selon l'une quelconque des revendications 1 à 5, dans lequel la tuberculose
(a) est provoquée par un agent choisi parmi *Mycobacterium tuberculosis, M. africanum, M. bovis, M. bovis BCG, M. canetti, M. caprae, M. microti, M. mungi, M. orygis, M. pinnipedii, M. suricattae, M. kansasii, M. xenopi, M. scrofulaceum, M. abscessus, M. haemophilum* et *M. ulcerans* ; et/ou
(b) est une tuberculose pharmacorésistante, éventuellement dans lequel (i) la tuberculose pharmacorésistante résiste à au moins l'un parmi l'isoniazide, la rifampicine, la bédaquiline, le délamanide, le pyrazinamide et l'éthambutol ; ou (ii) la tuberculose est une tuberculose multirésistante et résiste à au moins l'un parmi l'isoniazide ou la rifampicine et à au moins un autre agent anti-mycobactérien.

7. Antagoniste du récepteur de l'interféron de type 1 pour une utilisation selon l'une quelconque des revendications précédentes, l'antagoniste du récepteur de l'interféron de type 1 étant administré par administration intraveineuse, intramusculaire, intrapéritonéale, intracérébrospinale, sous-cutanée, intra-articulaire, intrasynoviale, intrathécale, orale ou topique ou par inhalation, éventuellement l'antagoniste du récepteur de l'interféron de type 1 étant administré par administration intraveineuse ou sous-cutanée.

8. Antagoniste du récepteur de l'interféron de type 1 pour une utilisation selon l'une quelconque des revendications précédentes, l'antagoniste du récepteur de l'interféron de type 1 étant administré à une posologie d'environ 0,01 mg/kg à environ 100 mg/kg de poids corporel du sujet, éventuellement l'antagoniste du récepteur de l'interféron de type 1 étant administré à une posologie d'environ 10 mg/kg à environ 30 mg/kg de poids corporel du sujet.

9. Antagoniste du récepteur de l'interféron de type 1 pour une utilisation selon la revendication 8, l'antagoniste du récepteur de l'interféron de type 1 étant administré à une posologie de :
a. environ 0,3 mg/kg de poids corporel,
b. environ 1 mg/kg de poids corporel ;
c. environ 3 mg/kg de poids corporel ; ou
d. environ 10 mg/kg de poids corporel.

10. Antagoniste du récepteur de l'interféron de type 1 pour une utilisation selon la revendication 8 ou la revendication 9, l'antagoniste du récepteur de l'interféron de type 1 étant administré à une posologie de :
a. environ 10 mg/kg de poids corporel ;
b. environ 15 mg/kg de poids corporel ;
c. environ 20 mg/kg de poids corporel ;
d. environ 25 mg/kg de poids corporel ; ou
e. environ 30 mg/kg de poids corporel.

11. Antagoniste du récepteur de l'interféron de type 1 pour une utilisation selon l'une quelconque des revendications précédentes, l'antagoniste du récepteur de l'interféron de type 1 étant administré à une posologie fixe d'environ 50 mg à environ 2000 mg, éventuellement l'antagoniste du récepteur de l'interféron de type 1 étant administré à une posologie de :
a. environ 100 mg ;
b. environ 200 mg ;
c. environ 300 mg ;
d. environ 600 mg ;
e. environ 1000 mg ;
f. environ 1200 mg ;
g. environ 1500 mg ; ou
h. environ 2000 mg.

12. Antagoniste du récepteur de l'interféron de type 1 pour une utilisation selon l'une quelconque des revendications précédentes, l'antagoniste du récepteur de l'interféron de type 1 étant administré :
a. sous forme d'une dose unique ;
b. en deux doses ou plus une fois par semaine ;
c. une fois toutes les deux semaines ;
d. une fois toutes les trois semaines ;
e. une fois toutes les quatre semaines ;
f. une fois par mois ;
g. une fois tous les trois mois ; ou
h. une fois tous les six mois.

13. Antagoniste du récepteur de l'interféron de type 1 pour une utilisation selon l'une quelconque des revendications précédentes, l'antagoniste du récepteur de l'interféron de type 1 étant administré à des intervalles de un jour à six mois, éventuellement l'antagoniste du récepteur de l'interféron de type 1 étant administré à des intervalles de :
a. une semaine ;
b. deux semaines ;
c. trois semaines ;
d. quatre semaines ;
e. un mois ;
f. deux mois ;
g. trois mois ;
h. quatre mois ;
i. cinq mois ; ou
j. six mois.

14. Antagoniste du récepteur de l'interféron de type 1 pour une utilisation selon l'une quelconque des revendications précédentes, l'antagoniste du récepteur de l'interféron de type 1 étant administré par voie intraveineuse ou sous-cutanée à des doses de 1 mg/kg à 30 mg/kg de poids corporel, à des intervalles de 1 semaine à 4 semaines.

15. Antagoniste du récepteur de l'interféron de type 1 pour une utilisation selon l'une quelconque des revendications précédentes, le procédé comprenant en outre l'administration, au sujet, d'une quantité thérapeutiquement efficace d'un agent thérapeutique additionnel, éventuellement dans lequel l'agent thérapeutique additionnel est :
(a) un agent antimicrobien ou antibactérien, éventuellement l'agent antimicrobien ou antibactérien étant choisi dans la liste comprenant l'isoniazide, la rifampicine, la bédaquiline, le délamanide, le pyrazinamide et l'éthambutol ;
(b) un agent anti-kinétoplastidés, choisi parmi le stibogluconate de sodium, l'antimoniate de méglumine, l'amphotéricine B liposomale, la miltéfosine, la pentamidine ou les antibiotiques ;
(c) un agent anti-kinétoplastidés choisi parmi la pentamidine, la suramine, le mélarsoprol, l'éflornithine ou le nifurtimox ; ou
(d) un agent antibiotique choisi parmi la streptomycine, la gentamicine, l'amikacine, le chloramphénicol, la tétracycline, l'érythromycine ou les fluoroquinolones.
